(19) Europäisches Patentamt
European Patent Office
Office européen des brevets

(11) Veröffentlichungsnummer: **0 268 951**
**A2**

(12) # EUROPÄISCHE PATENTANMELDUNG

(21) Anmeldenummer: **87116764.9**

(22) Anmeldetag: **13.11.87**

(51) Int. Cl.⁴: **C07D 487/04** , A01N 43/90 , C07D 249/14 , //(C07D487/04,249:00,239:00)

(30) Priorität: **25.11.86 DE 3640155**

(43) Veröffentlichungstag der Anmeldung: **01.06.88 Patentblatt 88/22**

(84) Benannte Vertragsstaaten: **BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Jelich, Klaus, Dr.**
**Pahlkestrasse 5**
**D-5600 Wuppertal 1(DE)**
Erfinder: **Krämer, Wolfgang, Dr.**
**Rosenkranz 25**
**D-5093 Burscheid 2(DE)**
Erfinder: **Santel, Hans-Joachim, Dr.**
**Grünstrasse 9a**
**D-5090 Leverkusen(DE)**
Erfinder: **Schmidt, Robert R., Dr.**
**Im Waldwinkel 110**
**D-5060 Bergisch-Gladbach 2(DE)**
Erfinder: **Strang, Robert Harry, Dr.**
**Unterdofstrasse 6A**
**D-4000 Düsseldorf 31(DE)**

(54) **Triazolo-pyrimidin-2-sulfonamide.**

(57) Die Erfindung betrifft neue Triazolo-pyrimidin-2-sulfonamide der Formel (I),

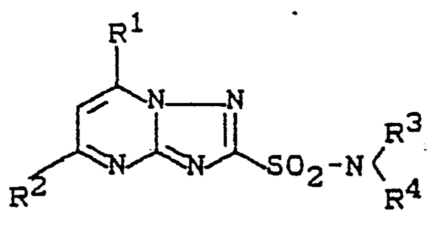

(I)

in welcher
$R^1$ für einen Rest $-CH_2-O-R^5$ steht und gleichzeitig $R^2$ für Alkyl steht oder
$R^2$ für einen Rest $-CH_2-O-R^5$ steht und gleichzeitig $R^1$ für Alkyl steht,
$R^3$ für Wasserstoff, Alkyl, Alkylcarbonyl, Alkoxycarbonyl, Alkylsulfonyl, Alkenyl, Alkinyl oder für gegebenenfalls substituiertes Aralkyl steht und
$R^4$ für jeweils gegebenenfalls substituiertes Aryl oder Heteroaryl steht und
$R^5$ für Alkyl steht,
mehrere Verfahren und neue Zwischenprodukte zu ihrer Herstellung sowie ihre Verwendung als Herbizide.

EP 0 268 951 A2

## Triazolo-pyrimidin-2-sulfonamide

Die Erfindung betrifft neue Triazolo-pyrimidin-2-sulfonamide, mehrere Verfahren und neue Zwischenprodukte zu ihrer Herstellung sowie ihre Verwendung als Herbizide.

Es ist bereits bekannt, daß bestimmte Triazolo-pyrimidin-2-sulfonamid-Derivate, wie z.B. 2,6-Dimethyl-N-(2-methoxycarbonylphenyl)-1-,2,4-triazolo-[1,5-a]-pyrimidin-2-sulfonamid herbizide Eigenschaften aufweisen (vgl. z.B. EP-A 142 152).

Die herbizide Wirksamkeit dieser vorbekannten Verbindungen gegenüber bestimmten Problemunkräutern ist jedoch ebenso wie ihre Verträglichkeit gegenüber bestimmten Kulturpflanzen nicht immer völlig zufriedenstellend.

Es wurden neue Triazolo-pyrimidin-2-sulfonamide der allgemeinen Formel (I),

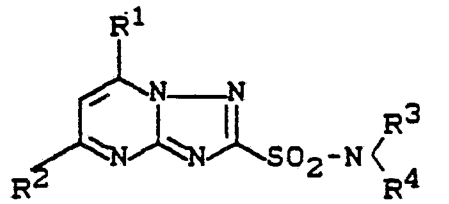

(I)

in welcher
$R^1$ für einen Rest $-CH_2-O-R^5$ steht und gleichzeitig $R^2$ für Alkyl steht oder
$R^2$ für einen Rest $-CH_2-O-R^5$ steht und gleichzeitig $R^1$ fur Alkyl steht,
$R^3$ für Wasserstoff, Alkyl, Alkylcarbonyl, Alkoxycarbonyl, Alkylsulfonyl, Alkenyl, Alkinyl oder für gegebenenfalls substituiertes Aralkyl steht,
$R^4$ für jeweils gegebenenfalls substituiertes Aryl oder Heteroaryl steht und
$R^5$ für Alkyl steht,
gefunden.

Weiterhin wurde gefunden, daß man die neuen Triazolopyrimidin-2-sulfonamide der allgemeinen Formel (I),

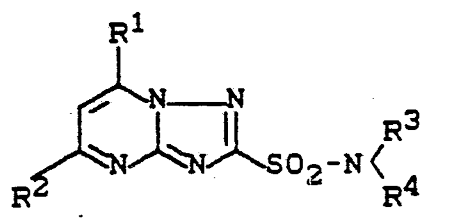

(I)

in welcher
$R^1$ für einen Rest $-CH_2-O-R^5$ steht und gleichzeitig $R^2$ für Alkyl steht oder
$R^2$ für einen Rest $-CH_2-O-R^5$ steht und gleichzeitig $R^1$ fur Alkyl steht,
$R^3$ für Wasserstoff, Alkyl, Alkylcarbonyl, Alkoxycarbonyl, Alkylsulfonyl, Alkenyl, Alkinyl oder für gegebenenfalls substituiertes Aralkyl steht und
$R^4$ für jeweils gegebenenfalls substituiertes Aryl oder Heteroaryl steht und
$R^5$ für Alkyl steht,
nach einem der im folgenden beschriebenen Verfahren erhält:

(a) Man erhält Triazolo-pyrimidin-2-sulfonamide der Formel (Ia),

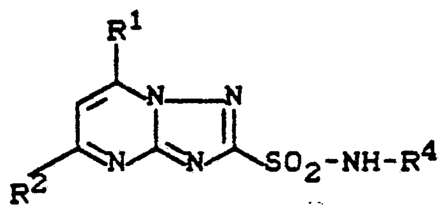

(Ia)

in welcher
$R^1$, $R^2$, und $R^4$ die oben angegebene Bedeutung haben,
wenn man Amino-triazolylsulfonamide der Formel (II),

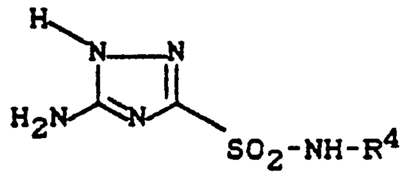

(II)

in welcher
$R^4$ die oben angegebene Bedeutung hat,
mit 1,3-Diketonen der Formel (III),

$$R^6-\overset{O}{\overset{\|}{C}}-CH_2-\overset{O}{\overset{\|}{C}}-CH_2-O-R^5 \quad \text{(III)}$$

in welcher
$R^5$ die oben angegebenen Bedeutung hat und
$R^6$ für Alkyl steht,
gegebenenfalls in Gegenwart eines Verdünnungsmittels umsetzt; oder

(b) man erhält Triazolo-pyrimidin-2-sulfonamide der Formel (Ib),

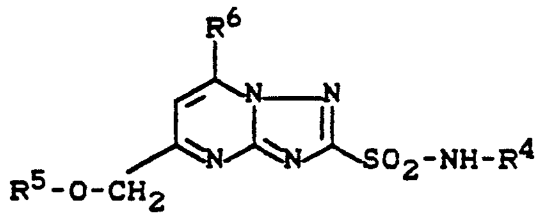

(Ib)

in welcher
$R^4$ und $R^5$ die oben angegebene Bedeutung haben und
$R^6$ für Alkyl steht,
wenn man Triazolo-pyrimidin-2-sulfochloride der Formel (IV),

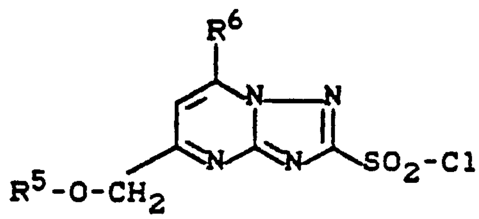

(IV)

in welcher
$R^5$ und $R^6$ die oben angegebene Bedeutung haben,
mit Aminen der Formel (V),

$R^4$-$NH_2$ (V)

in welcher
$R^4$ die oben angegebene Bedeutung hat,
gegebenenfalls in Gegenwart eines Verdünnungsmittels und gegebenenfalls in Gegenwart eines Säurebindemittels umsetzt; oder

(c) man erhält Triazolo-pyrimidin-2-sulfonamide der Formel (Ic),

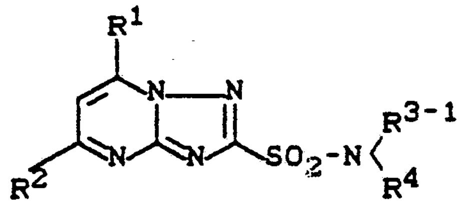

(Ic)

in welcher
$R^1$, $R^2$, und $R^4$ die oben angegebene Bedeutung haben und
$R^{3-1}$ für Alkyl, Alkenyl, Alkinyl, für gegebenenfalls substituiertes Aralkyl, für Alkylcarbonyl, Alkoxycarbonyl oder Alkylsulfonyl steht,
wenn man die nach Verfahren (a) oder (b) erhältlichen Triazolo-pyrimidin-2-sulfonamide der Formel (Ia)

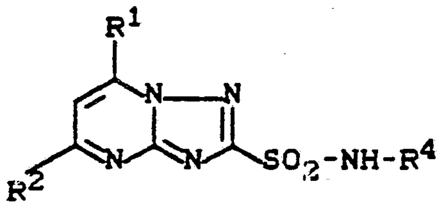

(Ia)

in welcher
$R^1$, $R^2$ und $R^4$ die oben angegebene Bedeutung haben,
mit Alkylierungs-, Acylierungs-oder Sulfonylierungsmitteln der Formel (VI),
$R^{3-1}$-Y (VI),
in welcher
$R^{3-1}$ die oben angegebene Bedeutung hat und
Y für eine elektronenanziehende Abgangsgruppe steht,
gegebenenfalls in Gegenwart eines Verdünnungsmittels und gegebenenfalls in Gegenwart einer Base umsetzt.

Schließlich wurde gefunden, daß die neuen Triazolo-pyrimidin-2-sulfonamide der allgemeinen Formel (I) herbizide Eigenschaften besitzen.

Überraschenderweise zeigen die erfindungsgemäßen Triazolo-pyrimidin-2-sulfonamide der allgemeinen Formel (I) eine erheblich bessere herbizide Wirksamkeit gegen Problemunkräuter als die aus dem Stand der Technik bekannten Triazolo-pyrimidin-2-sulfonamid-Derivate, wie beispielsweise das 2,6-Dimethyl-N-(2-methoxycarbonylphenyl)-1,2,4-triazolo-[1,5-a]-pyrimidin-2-sulfonamid, welche chemisch und wirkungsmäßig naheliegende Verbindungen sind.

Die erfindungsgemäßen Triazolo-pyrimidin-2-sulfonamide sind durch die Formel (I) allgemein definiert. Bevorzugt sind Verbindungen der Formel (I), bei welchen
$R^1$ für einen Rest $-CH_2-O-R^5$ steht und gleichzeitig $R^2$ für geradkettiges oder verzweigtes Alkyl mit 1 bis 6 Kohlenstoffatomen steht oder
$R^2$ für einen Rest $-CH_2-O-R^5$ steht und gleichzeitig $R^1$ für geradkettiges oder verzweigtes Alkyl mit 1 bis 6 Kohlenstoffatomen steht, wobei
$R^5$ in beiden Fällen jeweils für geradkettiges oder verzweigtes Alkyl mit 1 bis 6 Kohlenstoffatomen steht,
$R^3$ für Wasserstoff, für jeweils geradkettiges oder verzweigtes Alkyl, Alkylcarbonyl, Alkoxycarbonyl oder Alkylsulfonyl mit jeweils 1 bis 4 Kohlenstoffatomen in den einzelnen Alkylteilen, für jeweils geradkettiges oder verzweigtes Alkenyl oder Alkinyl mit jeweils 3 bis 6 Kohlenstoffatomen oder für im Alkylteil geradkettiges oder verzweigtes und im Arylteil gegebenenfalls einfach oder mehrfach, gleich oder verschieden substituiertes Aralkyl mit 1 bis 4 Kohlenstoffatomen im Alkylteil und 6 bis 10 Kohlenstoffatomen im Arylteil steht, wobei als Arylsubstituenten infrage kommen: Fluor, Chlor, Brom, Iod, Cyano, Nitro, Hydroxy, sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen und
$R^4$ für gegebenenfalls einfach oder mehrfach, gleich oder verschiedenen substituiertes Aryl mit 6 bis 10 Kohlenstoffatomen oder für einen gegebenenfalls einfach oder mehrfach, gleich oder verschieden substituierten und/oder benzannellierten 5-bis 7-gliedrigen Heterocyclus mit 1 bis 3 Heteroatomen, insbesondere Stickstoff, Sauerstoff und/oder Schwefel steht, wobei als Substituenten jeweils infrage kommen: Fluor, Chlor,

Brom, Iod, Cyano, Nitro, Hydroxy, jeweils geradkettiges oder verzweigtes Alkyl, Alkoxy, Alkylthio, Alkylcarbonyl, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl, Halogenalkylsufonyl, Halogenalkylcarbonyl oder Halogenalkoxycarbonyl mit jeweils 1 bis 6 Kohlenstoffatomen und 1 bis 9 gleichen oder verschiedenen Halogenatomen, Phenyl, Phenoxy, Phenylthio, Phenylcarbonyl, Hydroxycarbonyl, jeweils geradkettiges oder verzweigtes Alkoxycarbonyl, Alkenyloxycarbonyl oder Alkoxyalkoxycarbonyl mit jeweils 1 bis 6 Kohlenstoffatomen in den einzelnen Alkylteilen bzw. 3 bis 6 Kohlenstoffatomen im Alkenylteil, sowie Hydroximinoalkyl oder geradkettiges oder verzweigtes Alkoximinoalkyl mit jeweils 1 bis 4 Kohlenstoffatomen in den einzelnen Alkylteilen.

Besonders bevorzugt sind Verbindungen der allgemeinen Formel (I), bei welchen

$R^1$ für einen Rest $-CH_2-O-CH_3$ oder $-CH_2-O-C_2H_5$ steht und gleichzeitig $R^2$ für Methyl oder Ethyl steht oder

$R^2$ für einen Rest $-CH_2-O-CH_3$ oder $-CH_2-O-C_2H_5$ steht und gleichzeitig $R^1$ für Methyl oder Ethyl steht,

$R^3$ für Wasserstoff, Methyl, Ethyl, Acetyl, Methoxycarbonyl, Ethoxycarbonyl, Methylsulfonyl, Allyl, Propargyl oder Benzyl steht und

$R^4$ für jeweils gegebenenfalls ein-bis dreifach, gleich oder verschieden substituiertes Phenyl oder Naphthyl oder für einen gegebenenfalls ein-bis dreifach gleich oder verschieden substituierten und/oder benzannellierten Heterocyclus der Formel

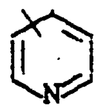 ; 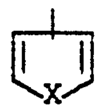 ; 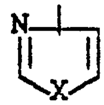 ; 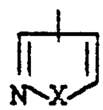 ; 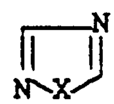

oder 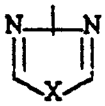

steht,

wobei X jeweils für Sauerstoff, Schwefel oder eine NH-Gruppe oder $NCH_3$-Gruppe steht und wobei als Substituenten jeweils infrage kommen, Fluor, Chlor, Brom, Iod, Cyano, Nitro, Hydroxy, Methyl, Ethyl, n-oder i-Propyl, n-, i-, s-oder t-Butyl, Methoxy, Ethoxy, n-oder i-Propoxy, Methylthio, Ethylthio, Acetyl, Propionyl, Methylsulfinyl, Methylsulfonyl, Trifluoromethyl, Trifluormethoxy, Trifluormethylthio, Trifluormethylsulfinyl, Trifluormethylsulfonyl, Chloracetyl, Dichloracetyl, Trifluoracetyl, Chlorethoxycarbonyl, Phenyl, Phenoxy, Phenylthio, Benzoyl, Hydroxycarbonyl, Methoxycarbonyl, Ethoxycarbonyl, n-oder i-Propoxycarbonyl, Allyloxycarbonyl, Methoxymethoxycarbonyl, Ethoxyethoxycarbonyl, Hydroximinomethyl, Methoximinomethyl, Methoximinoethyl und Ethoximinoethyl.

Ganz besonders bevorzugt sind Verbindungen der allgemeinen Formel (I), bei welchen

$R^1$ für einen Methoxymethylrest steht und gleichzeitig $R^2$ für Methyl steht oder

$R^2$ für einen Methoxymethylrest steht und gleichzeitig $R^1$ für Methyl steht,

$R^3$ für Wasserstoff steht und

$R^4$ für jeweils gegebenenfalls ein-bis dreifach, gleich oder verschieden substituiertes Phenyl, α-Naphthyl oder β-Naphthyl steht oder für einen gegenbenenfalls ein-bis dreifach gleich oder verschieden substituierten und/oder benzannellierten Heterocyclus der Formel

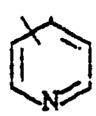 ; 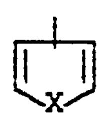 ; 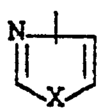 oder 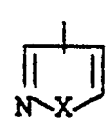

steht,

wobei X jeweils für Sauerstoff, Schwefel oder eine NH-Gruppe oder $NCH_3$-Gruppe steht und wobei als Substituenten jeweils infrage kommen: Fluor, Chlor, Brom, Iod, Cyano, Nitro, Hydroxy, Methyl, Ethyl, Methoxy, Methylthio, Acetyl, Chlorethoxycarbonyl, Trifluormethyl,Trifluormethoxy, Trifluormethylthio, Phenyl, Phenoxy, Phenylthio, Hydroxycarbonyl, Methoxycarbonyl, Ethoxycarbonyl, Allyloxycarbonyl, Ethoxyethoxycarbonyl, Hydroximinomethyl, Methoximinomethyl oder Methoximinoethyl.

Im einzelnen seien außer den bei Herstellungsbeispielen genannten Verbindungen die folgenden Triazolopyrimidin-2-sulfonamide der allgemeinen Formel (I) genannt

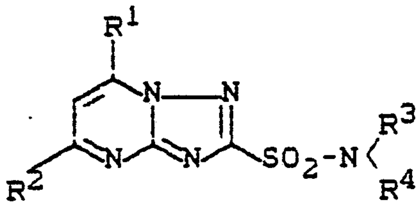

(I)

| $R^1$ | $R^2$ | $-N\langle^{R^3}_{R^4}$ |
|---|---|---|
| $CH_3O-CH_2-$ | $CH_3$ | 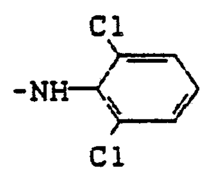 |
| $CH_3$ | $CH_3O-CH_2-$ | 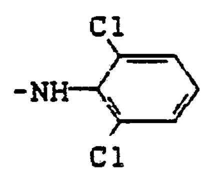 |
| $CH_3O-CH_2-$ | $CH_3$ | 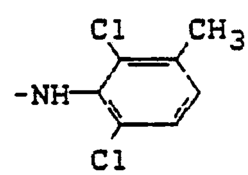 |

| $R^1$ | $R^2$ | 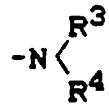 |
|---|---|---|
| $CH_3$ | $CH_3O-CH_2-$ | 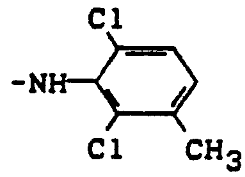 |
| $CH_3O-CH_2-$ | $CH_3$ | 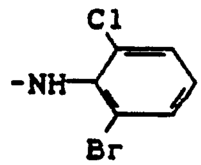 |
| $CH_3$ | $CH_3O-CH_2-$ | 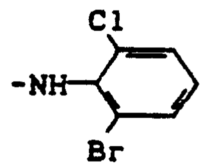 |
| $CH_3O-CH_2-$ | $CH_3$ | 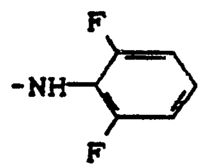 |
| $CH_3$ | $CH_3O-CH_2-$ | 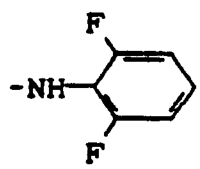 |
| $CH_3O-CH_2-$ | $CH_3$ | 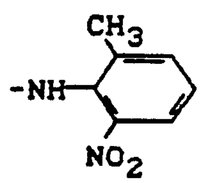 |
| $CH_3$ | $CH_3O-CH_2-$ | 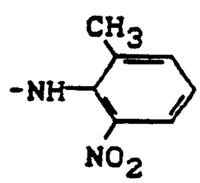 |

| $R^1$ | $R^2$ | 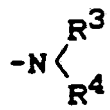 |
|---|---|---|
| $CH_3O-CH_2-$ | $CH_3$ | 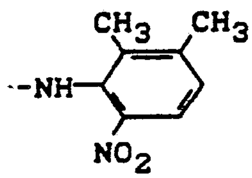 |
| $CH_3$ | $CH_3O-CH_2-$ | 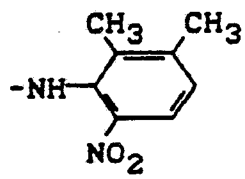 |
| $CH_3O-CH_2-$ | $CH_3$ | 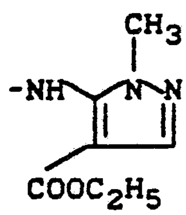 |
| $CH_3$ | $CH_3O-CH_2-$ | 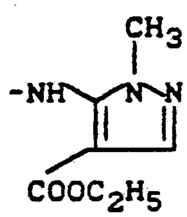 |
| $CH_3-O-CH_2-$ | $CH_3$ | 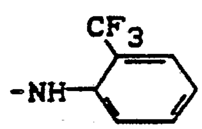 |
| $CH_3$ | $CH_3-O-CH_2-$ | 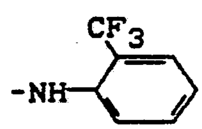 |
| $CH_3-O-CH_2-$ | $CH_3$ | 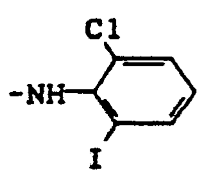 |

| $R^1$ | $R^2$ | $-N\langle^{R^3}_{R^4}$ |
|---|---|---|
| $CH_3$ | $CH_3-O-CH_2-$ | 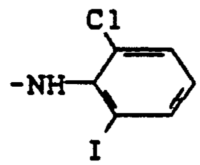 |
| $CH_3$ | $CH_3-O-CH_2-$ | 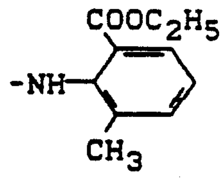 |
| $CH_3-O-CH_2-$ | $CH_3$ | 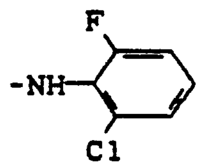 |
| $CH_3-O-CH_2-$ | $CH_3$ | 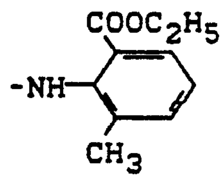 |
| $CH_3-O-CH_2-$ | $CH_3$ | 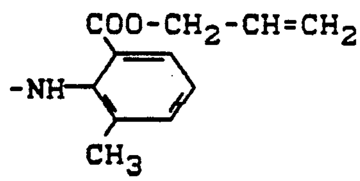 |
| $CH_3$ | $CH_3-O-CH_2-$ | 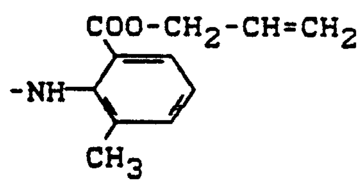 |
| $CH_3-O-CH_2-$ | $CH_3$ | 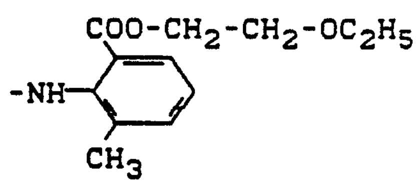 |

| $R^1$ | $R^2$ | $-N{<}^{R^3}_{R^4}$ |
|---|---|---|
| $CH_3$ | $CH_3-O-CH_2-$ | 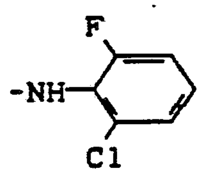 |
| $CH_3$ | $CH_3-O-CH_2-$ | 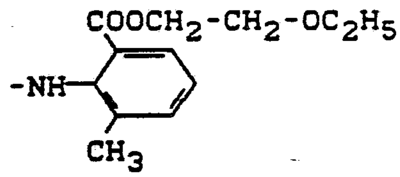 |
| $CH_3$ | $CH_3-O-CH_2-$ | 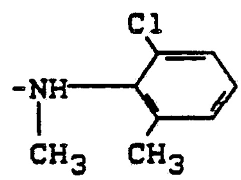 |
| $CH_3$ | $CH_3-O-CH_2-$ | 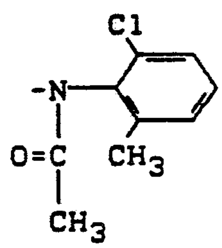 |
| $CH_3$ | $CH_3-O-CH_2-$ | 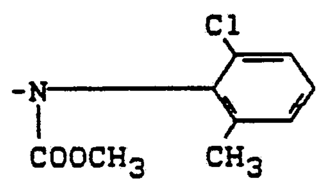 |
| $CH_3$ | $CH_3-O-CH_2-$ | 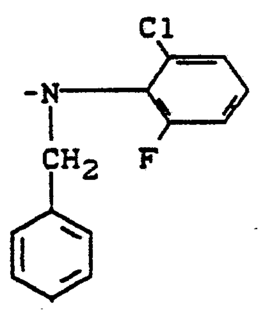 |

| $R^1$ | $R^2$ | $-N \begin{smallmatrix} R^3 \\ R^4 \end{smallmatrix}$ |
|---|---|---|
| $CH_3$ | $CH_3-O-CH_2-$ | 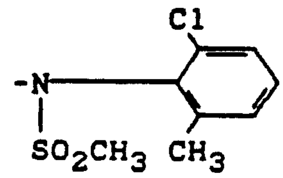 |
| $CH_3$ | $CH_3-O-CH_2-$ | 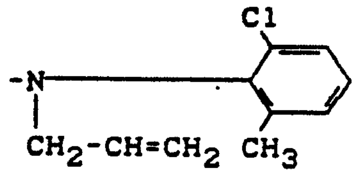 |
| $CH_3$ | $CH_3-O-CH_2-$ | 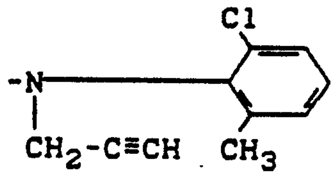 |

Verwendet man beispielsweise 5-Amino-3-(2,6-dichlorphenylaminosulfonyl)-1,2,4-triazol und Methoxyacetylaceton als Ausgangsstoffe, so läßt sich der Reaktionsablauf des erfindungsgemäßen Verfahrens (a) durch das folgende Formelschema darstellen

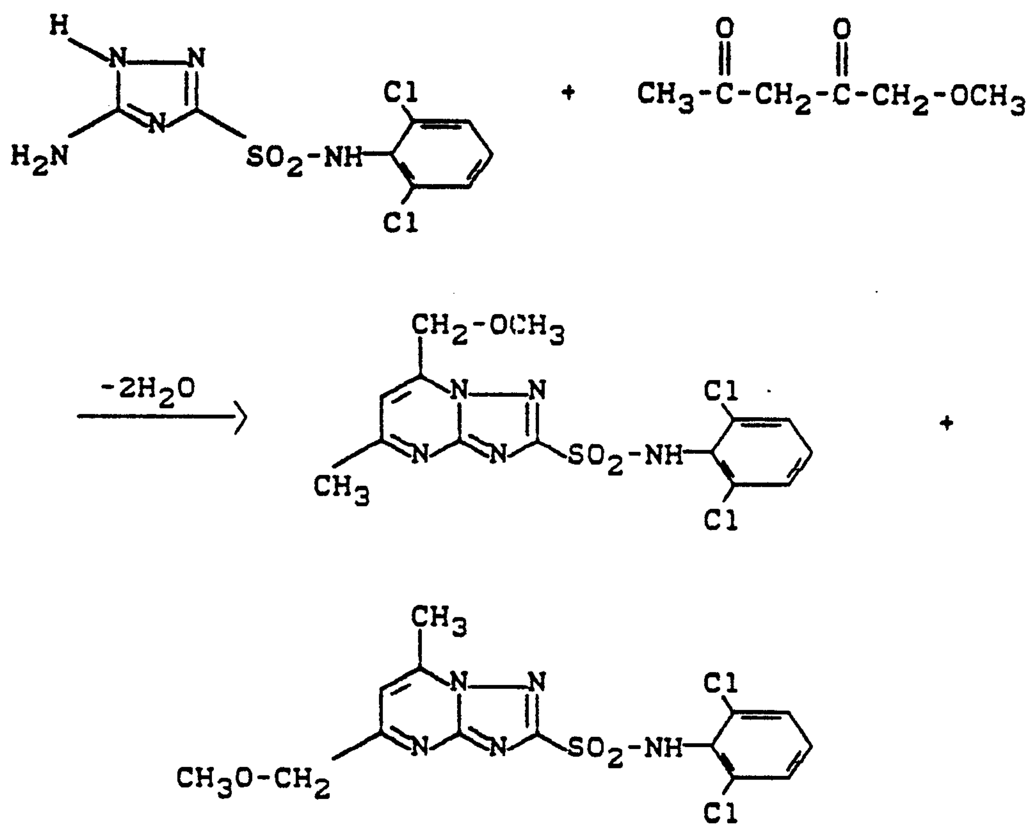


Verwendet man beispielsweise 5-Methoxymethyl-7-methyl-1,2,4-triazolo-[1,5-a]-pyrimidin-2-sulfonylchlorid und 2-Chlor-6-methylanilin als Ausgangsstoffe, so läßt sich der Reaktionsablauf des erfindungsgemäßen Verfahrens (b) durch das folgende Formelschema darstellen:

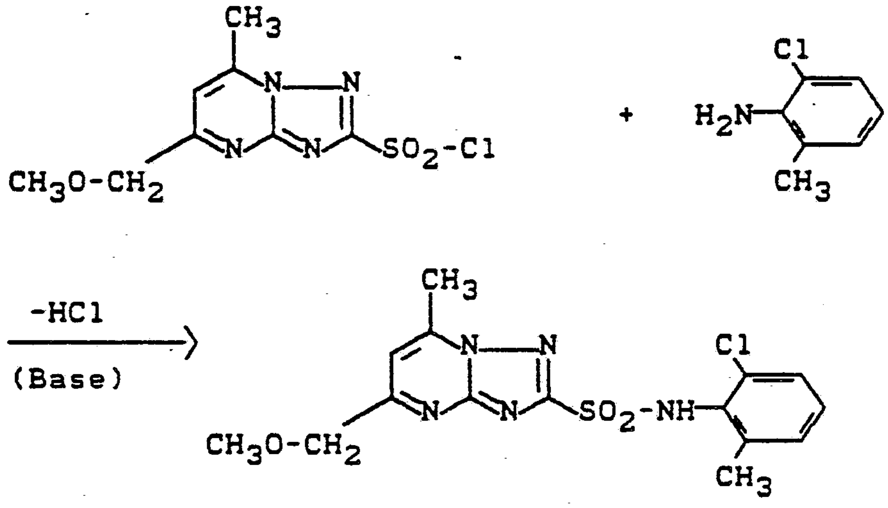


Verwendet man beispielsweise 5-Methyl-7-methoxymethyl-1,2,4-triazolo-[1,5-a]-pyrimidin-2-[N-(2,6-dimethylphenyl)]-sulfonamid und Methyliodid als Ausgangsstoffe, so läßt sich der Reaktionsabluaf des erfindungsgemäßen Verfahrens (c) durch das folgende Formelschema darstellen

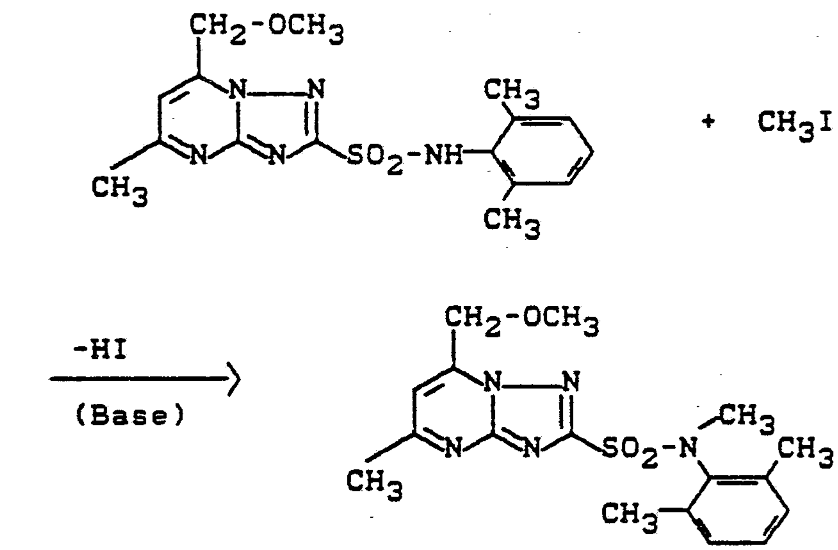


Die zur Durchführung des erfindungsgemäßen Verfahrens (a) als Ausgangsstoffe benötigten Aminotriazolylsulfonamide sind durch die Formel (II) allgemein definiert. In dieser Formel (II) steht $R^4$ vorzugsweise bzw. besonders bevorzugt für diejenigen Reste, die bereits im Zusammenhang mit der Beschreibung der erfindungsgemäßen Stoffe der Formel (I) als bevorzugt bzw. besonders bevorzugt für diesen Substituenten genannt wurden. In der Formel (II) steht $R^4$ ganz besonders bevorzugt für diejenigen Reste, die im Zusammenhang mit der Beschreibung der erfindungsgemäßen Stoffe der Formel (I) als ganz besonders bevorzugt für diese Substituenten genannt wurden.

Die Aminotriazolylsulfonamide der Formel (II) sind noch nicht bekannt.

Man erhält sie, wenn man 3-Amino-5-benzylthio-1,2,4-triazol der Formel (VIIa),

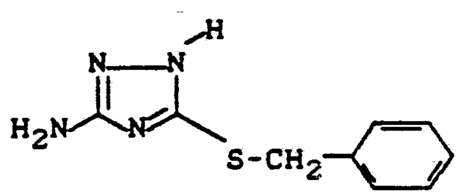

(VIIa)

(vgl. z.B. J. Heterocycl. Chem. 12, 1187 [1975]; EP 142 152) welches im tautomeren Gleichgewicht vorliegt mit der enstprechenden 5-Amino-3-benzylthio-Verbindungen der Formel (VIIb)

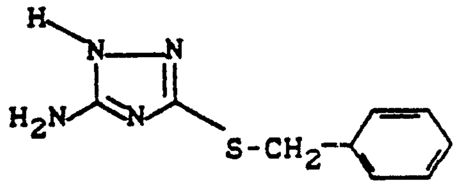

(VIIb)

zunächst in einer 1. Stufe mit Chlorameisensäurephenylester in Gegenwart eines Verdünnungsmittels wie beispielsweise Pyridin bei Temperaturen zwischen -20 °C und + 20 °C umsetzt und das so erhältliche 1-Phenoxycarbonyl-3-benzylthio-5-amino-1,2,4-triazol der Formel (VIII)

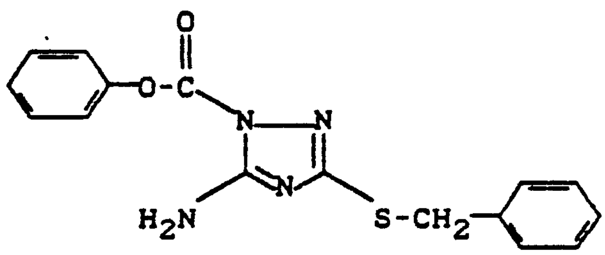

(VIII)

in einer 2. Stufe mit elementarem Chlor in Gegenwart von Wasser und in Gegenwart eines Verdünnungsmittels, wie beispielsweise Chloroform sowie in Gegenwart eines Reaktionshilfmittels wie beispielsweise Eisessig bei Temperaturen zwischen -20 °C und + 20 °C umsetzt und das derart erhältliche 5-Amino-1-phenoxycarbonyl-1,2,4-triazol-3-yl-sulfonylchlorid der Formel (IX)

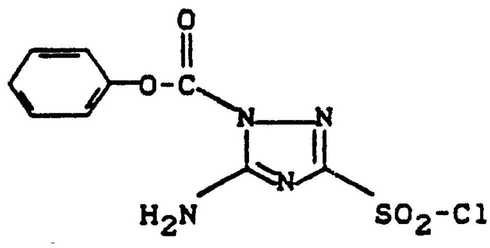

(IX)

in einer 3. Stufe mit Aminen Der Formel (V),

$R^4$-$NH_2$ (V)

in welcher

$R^4$ die oben angegebene Bedeutung hat,

gegebenenfalls in Gegenwart eines Verdünnungsmittels wie beispielsweise Dichlormethan sowie in Gegenwart eines Säurebindemittels wie beispielsweise Pyridin und gegebenenfalls in Gegenwart eines Reaktionshilfsmittels wie beispielsweise 4-Dimethylaminopyridin bei Temperaturen zwischen 0 °C und 60 °C umsetzt und anschließend in einer 4. Stufe aus den so erhältlichen 5-Amino-1-phenoxycarbonyl-1,2,4-triazol-3-yl-sulfonamiden der Formel (X),

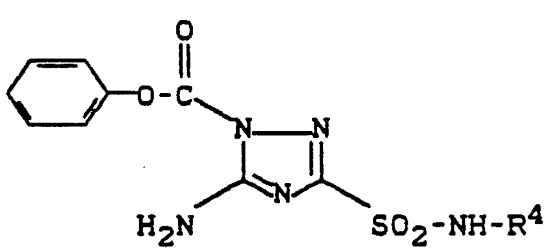

(X)

in welcher

$R^4$ die oben angegebene Bedeutung hat,

mit wässriger Natronlauge gegebenenfalls in Gegenwart eines Verdünnungsmittels wie beispielsweise Ethanol bei Temperaturen zwischen 0 °C und 40 °C die Phenoxycarbonyl-Schutzgruppe in der 1-Position des Triazolringes wieder abspaltet.

Die Verbindungen der Formeln (VIII), (IX) und (X) sind neu und ebenfalls Gegenstand der vorliegenden Erfindung. Sie besitzen ein gemeinsames Strukturelement und lassen sich in der Formel (XIV)

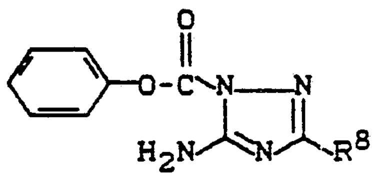

(XIV)

in welcher
$R^8$ für Benzylthio, $-SO_2Cl$ oder $-SO_2-NH-R^4$ steht, wobei
$R^4$ für jeweils gegebenenfalls substituiertes Aryl oder Heteroaryl steht.
zusammenfassen.

In den Formeln (X) und (XIV) steht $R^4$ vorzugsweise bzw. besonders bevorzugt bzw. ganz besonders bevorzugt für diejenigen Reste, die bereits bei der Beschreibung der erfindungsgemäßen Stoffe der Formel (I) als bevorzugt bzw. besonders bevorzugt bzw. ganz besonders bevorzugt für diesen Substituenten genannt wurden.

Die zur Durchführung des erfindungsgemäßen Verfahrens (a) weiterhin als Ausgangsstoffe benötigten 1,3-Diketone sind durch die Formel (III) allgemein definiert. In dieser Formel (III) steht $R^5$ vorzugsweise für diejenigen Reste, die bereits im Zusammenhang mit der Beschreibung der erfindungsgemäßen Stoffe der Formel (I) als bevorzugt für diesen Substituenten genannt wurden. $R^6$ steht vorzugsweise für geradkettiges oder verzweigtes Alkyl mit 1 bis 6 Kohlenstoffatomen, besonders für Methyl, Ethyl, n-oder i-Propyl sowie n-, i-, s-oder t-Butyl, insbesondere für Methyl oder Ethyl.

Die 1,3-Diketone der Formel (III) sind allgemein bekannte Verbindungen der organischen Chemie oder erhältlich nach allgemein bekannten Verfahren in Analogie zu bekannten Verbindungen (vgl. z.B. W. F. Bruce u. H. W. Coover, J. Am. Chem. Soc. 66, 2092 [1944]).

Die zur Durchführung des erfindungsgemäßen Verfahrens (b) als Ausgangsstoffe benötigten Triazolo-pyrimidin-2-sulfochloride sind durch die Formel (IV) allgemein definiert. In dieser Formel (IV) stehen $R^5$ un $R^6$ vorzugsweise jeweils für geradkettiges oder verzweigtes Alkyl mit 1 bis 6 Kohlenstoffatomen, besonders für Methyl, Ethyl, n-oder i-Propyl sowie n-, i-, s-, oder t-Butyl, insbesondere für Methyl oder Ethyl.

Die Triazolo-pyrimidin-2-sulfochloride der Formel (IV) sind noch nicht bekannt und Gegenstand der vorliegenden Erfindung.

Man erhält sie, wenn man 5-Amino-1,2,4-triazol-Derivate der Formel (VII),

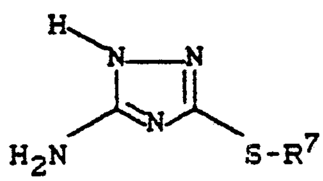

(VII)

in welcher
$R^7$ für Wasserstoff oder für einen Benzylrest steht,
mit 1,3-Diketonen der Formel (III),

$$R^6-\overset{O}{\overset{\|}{C}}-CH_2-\overset{O}{\overset{\|}{C}}-CH_2-OR^5 \quad (III)$$

in welcher
$R^5$ und $R^6$ die oben angegebene Bedeutung haben,
zunächst in einer 1. Stufe in Analogie zu dem erfindungsgemäßen Verfahren (a) gegebenenfalls in Gegenwart eines Verdünnungsmittels wie beispielsweise Eisesssig bei Temperaturen zwischen 20 °C und 120 °C kondensiert und das auf diese Weise erhältliche Gemische aus stellungsisomeren Triazolopyrimidin-Derivaten der Formeln (XIa) und (XIb),

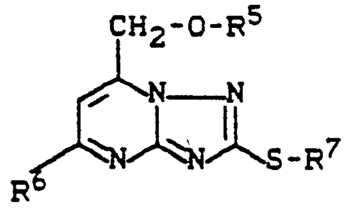

+

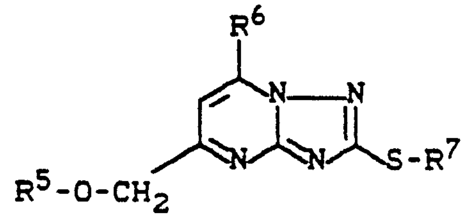

(XIa) (XIb)

in welcher

$R^5$, $R^6$ und $R^7$ die oben angegebene Bedeutung haben

in einer 2. Stufe mit elementarem Chlor in Gegenwart von Wasser sowie gegebenenfalls in Gegenwart eines Verdünnungsmittels wie beispielsweise Chloroform bei Temperaturen zwischen - 20 °C und + 20 °C umsetzt.

Das auf diese Weise erhältliche Gemisch aus gewünschtem Triazolo-pyrimidin-2-sulfochlorid der Formel (IV)

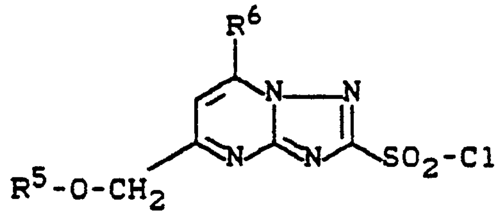

(IV)

und einer in 6-Stellung kernchlorierten Verbindung der Formel (XII),

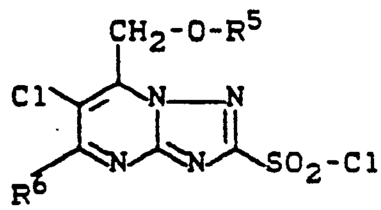

(XII)

wobei in beiden Formeln (IV) und (XII) $R^5$ und $R^6$ die oben angegebenen Bedeutungen haben, kann ohne weitere Auftrennung als Ausgangsverbindung zur Durchführung des erfindungsgemäßen Verfahrens (b) eingesetzt werden.

Die 5-Amino-1,2,4-triazol-Derivate der Formeln (VII), (VIIa) und VIIb) sind bekannt (vgl. z.B. EP 142 152).

Die Triazolopyrimidin-Derivate der Formeln (XIa) und (XIb) sind neu und Gegenstand der vorliegenden Erfindung. In diesen Formeln (XIa) und (XIb) stehen $R^5$ und $R^6$ vorzugsweise für geradkettiges oder verzweigtes Alkyl mit 1 bis 6 Kohlenstoffatomen, besonders für Methyl, Ethyl, n-oder i-Propyl oder n-, i-, s- oder t-Butyl, insbesondere für Methyl oder Ethyl.

Die Formeln (IV), (XIa) und (XIb) besitzen ein gemeinsames Strukturelement und lassen sich daher in der Formel (XV),

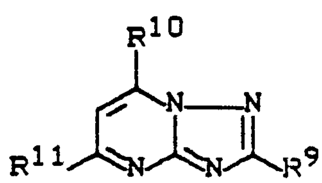

(XV)

in welcher

$R^9$ für $-SO_2Cl$ oder $-S-R^7$ steht, wobei

$R^7$ für Wasserstoff oder für einen Benzylrest steht,

$R^{10}$ für $R^6$ steht und

$R^{11}$ für $-CH_2-O-R^5$ steht oder
für den Fall, daß $R^9$ für $-S-R^7$ steht, $R^{10}$ auch für $-CH^2-O-R^5$ steht und $R^{11}$ für $R^6$ steht, wobei
$R^5$ und $R^6$ die oben angegebenen Bedeutung haben,
zusammenfassen.

Die zur Durchführung des erfindungsgemäßen Verfahrens (b) weiterhin als Ausgangsstoffe benötigten Amine sind durch die Formel (V) allgemein definiert. In dieser Formel (V) steht $R^4$ vorzugsweise für diejenigen Reste, die bereits im Zusammenhang mit der Beschreibung der erfindungsgemäßen Stoffe der Formel (I) als bevorzugt für diesen Substituenten genannt wurden.

Die Amine der Formel (V) sind allgemein bekannte Verbindungen der organischen Chemie.

Die zur Durchführung des erfindungsgemäßen Verfahrens (c) als Ausgangsstoffe benötigten Triazolo-pyrimidin-2-sulfonamide sind durch die Formel (Ia) allgemein definiert. In dieser Formel (Ia) stehen $R^1$, $R^2$, und $R^4$ vorzugsweise für diejenigen Reste, die bereits im Zusammenhang mit der Beschreibung der erfingunsgemäßen Stoffe der Formel (I) als bevorzugt für diese Substituenten genannt wurden.

Die Triazolo-pyrimidin-2-sulfonamide der Formel (Ia) sind erfindungsgemäße Verbindungen und erhältlich mit Hilfe der erfindungsgemäßen Verfahren (a) oder (b).

Die zur Durchführung des erfindungsgemäßen Verfahrens (c) weiterhin als Ausgangsstoffe benötigten Alkylierungs-Acylierungs-oder Sulfonylierungsmittel sind durch die Formel (IV) allgemein definiert. In dieser Formel (VI) steht $R^{3-1}$ vorzugsweise für jeweils geradkettiges oder verzweigtes Alkyl, Alkylcarbonyl, Alkoxycarbonyl oder Alkylsulfonyl mit jeweils 1 bis 4 Kohlenstoffatomen in den einzelnen Alkylteilen, für jeweils geradkettiges oder verzweigtes Alkenyl oder Alkinyl mit jeweils 3 bis 6 Kohlenstoffatomen oder für im Alkylteil geradkettiges oder verzweigtes und im Arylteil gegebenenfalls einfach oder mehrfach, gleich oder verschieden substituiertes Aralkyl mit 1 bis 4 Kohlenstoffatomen im Alkylteil und 6 bis 10 Kohlenstoffatomen im Arylteil wobei als Arylsubstituenten infrage kommen: Fluor, Chlor, Brom, Iod, Cyano, Nitro, Hydroxy sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen;

$R^{3-1}$ steht insbesondere für Methyl, Ethyl, Acetyl, Methoxycarbonyl, Ethoxycarbonyl, Methylsulfonyl, Allyl, Propargyl oder Benzyl. Y steht vorzugsweise für Halogen, insbesondere für Chlor, Brom oder Iod.

Die Alkylierungs-, Acylierungs-oder Sulfonylierungsmittel der Formel (VI) sind allgemein bekannte Verbindungen der organischen Chemie.

Als Verdünnungsmittel zur Durchführung des erfindungsgemäßen Verfahrens (a) kommen inerte organische Lösungsmittel infrage. Mit besonderem Vorzug verwendet man polare organische Lösungsmittel, beispielsweise höhersiedende Alkohole wie Ethylenglykolmonoethylether, Ethanol, Propanol oder Butanol oder Carbonsäuren wie beispielsweise Essigsäure.

Die Reaktionstemperaturen können bei der Durchführung des erfindungsgemäßen Verfahrens (a) in einem größeren Bereich variiert werden. Im allgemeinen arbeitet man bei Temperaturen zwischen 20 °C und 200 °C, vorzugsweise bei Temperaturen zwischen 50 °C und 150 °C.

Zur Durchführung des erfindungsgemäßen Verfahrens setzt man pro Mol an Aminotriazolylsulfonamid der Formel (II) im allgemeinen 1,0 bis 3,0 Mol, vorzugsweise 1,0 bis 2,0 Mol an 1,3-Diketon der Formel (III) ein. Die Reaktionsdurchführung, Aufarbeitung und Isolierung der Reaktionsprodukte der Formel (I) erfolgt in Analogie zu bekannten Verfahren (vgl. Z. B. EP 142 152). Dabei erhält man in der Regel Gemische aus den stellungsisomeren Triazolopyrimidin-2-sulfonamiden der Formeln (Ia1) und (Ib),

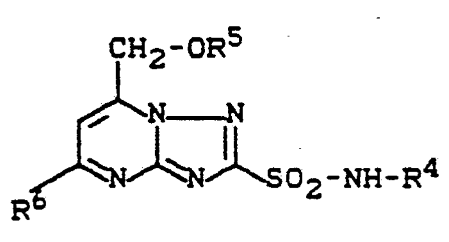

(Ia1)

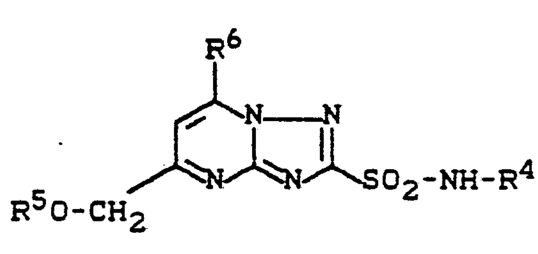

(Ib)

wobei
$R^4$, $R^5$ und $R^6$ jeweils die oben angegebene Bedeutung haben.

Diese Gemische lassen sich mit Hilfe üblicher Trennmethoden (Chromatographie, Kristallisation) in ihre Bestandteile trennen. Es ist jedoch auch möglich die Gemische als solche erfindungsgemäß zu verwenden.

Als Verdünnungsmittel zur Durchführung des erfindungsgemäßen Verfahrens (b) kommen ebenfalls inerte organische Lösungsmittel infrage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl-oder diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (b) wird vorzugsweise in Gegenwart eines geeigneten Säurebindemittels durchgeführt. Als solche kommen alle üblichen anorganischen oder organischen Basen in Frage. Hierzu gehören beispielsweise Alkalimetallhydroxide, wie Natriumhydroxdid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Es ist auch möglich, das als Reaktionspartner verwendete Amin der Formel (V) in entsprechendem Überschuß gleichzeitig als Säurebindemittel einzusetzen.

Die Reaktionstemperaturen können bei der Durchführung des erfindungsgemäßen Verfahrens (b) in einem größeren Bereich variiert werden. Im allgemeinen arbeitet man bei Temperaturen zwischen 0 °C und 150 °C, vorzugsweise bei Temperaturen zwischen 10 °C und 80 °C.

Zur Durchführung des erfindungsgemäßen Verfahrens (b) setzt man pro Mol an Triazolo-pyrimidin-2-sulfochlorid der Formel (IV) im allgemeinen 1,0 bis 5,0 Mol, vorzugsweise 1,0 bis 2,0 Mol an Amin der Formel (V) und gegebenenfalls 1,0 bis 2,0 Mol an Säurebindemittel ein. In einer bevorzugten Durchführungsform verwendet man das als Ausgangsprodukt infrage kommende Triazolo-pyrimidin-2-sulfochlorid der Formel (VI) in einem Rohproduktgemisch zusammen mit dem gleichzeitig entstandenen 6-Chlor-triazolo-pyrmidin-2-sulfochlorid der Formel (XII),

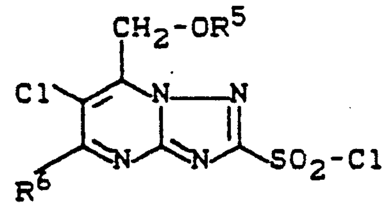

(XII)

(wobei $R^5$ und $R^6$ die oben angegebene Bedeutung haben) als Ausgangsverbindung. Die Auftrennung der entsprechenden Endprodukte der Formeln (Ib) und (XIII)

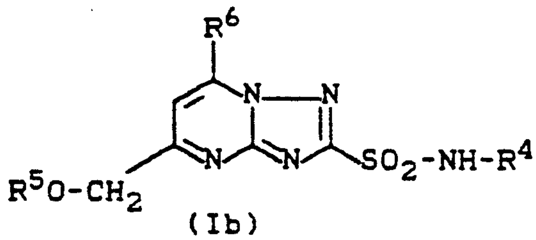

(Ib)

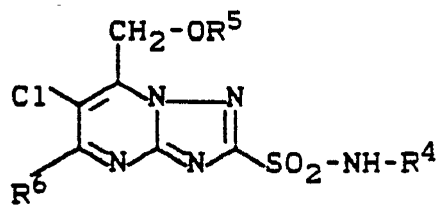

(XIII)

(wobei $R^4$, $R^5$ und $R^6$ jeweils die oben angegebene Bedeutung haben) erfolgt mit Hilfe üblicher Trennverfahren beispielsweise durch Chromatographie (vgl. hierzu auch die Herstellungsbeispiele).

Als Verdünnungsmittel zur Durchführung des erfindungsgemäßen Verfahrens (c) kommen ebenfalls inerte organische Lösungsmittel infrage. Vorugsweise verwendet man die bei Verfahren (b) genannten Lösungsmittel.

Das erfindungsgemäße Verfahren (c) wird vorzugsweise in Gegenwart eines basischen Reaktionshilfsmittels durchgeführt. Als solche kommen alle üblichen anorganischen oder organischen Basen infrage. Vorzugsweise verwendet man die bei Verfahen (b) genannten Hydroxide, Carbonate oder Amine.

Die Reaktionstemperaturen können bei der Durchführung des erfindungsgemäßen Verfahrens (c) in einem größeren Bereich variiert werden. Im allgemeinen arbeitet man bei Temperaturen zwischen - 20 °C und + 150 °C, vorzugsweise bei Temperaturen zwischen 0 °C und + 120 °C.

Zur Durchführung des erfindungsgemäßen Verfahrens (c) setzt man pro Mol an Triazolo-pyrimidin-2-sulfonamid der Formel (Ia) im allgemeinen 1,0 bis 5,0 Mol, vorzugsweise 1,0 bis 2,0 Mol an Alkylierungs-, Acylierungs-oder Sulfonylierungsmittel der Formel (VI) und gegebenenfalls 1,0 bis 5,0 Mol, vorzugsweise 1,0 bis 2,0 Mol an basischen Reaktionshilfsmittel ein. Die Reaktionsdurchführung, Aufarbeitung und

Isolierung der Reaktionsprodukte der Formel (Ic) erfolgt in Analogie zu allgemein üblichen Verfahren.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs aus diese Gattungen beschränkt, sondern erstreckt sich in gleicher Weise aus auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Die erfindungsgemäßen Verbindungen der Formel (I) eignen sich besonders zur Bekämpfung mono-und dikotyler Unkräuter im Vor-und im Nachauflaufverfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder - schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier-und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsufate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5,-triazin-2,4(1H,3H)-dion oder N-(2-Benzthiazolyl)-N,N'-dimethylharnstoff zur Unkrautbekämpfung in Getreide; 4-Amino-3-methyl-6-phenyl-1,2,4,-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage.

Auch Mischungen mit N,N-Dimethyl-N'-(3-chlor-4-methylphenyl)-harnstoff;
N,N-Dimethyl-N'-(4-isopropylphenyl)-harnstoff;
2-Chlor-4-ethylamino-6-isopropylamino-1,3,5-triazin;
4-Ethylamino-2-t-butylamino-6-methylthio-s-triazin;
2-Chlor-4-ethylamino-6-(3-cyanopropylamino)-1,3,5-triazin;
4-Amino-6-t-butyl-3-ethylthio-1,2,4,-triazin-5(4H)-on;
N-Methyl-2-(1,3-benzthiazol-2-yloxy)-acetanilid;
Chloressigsäure-N-(methoxymethyl)-2,6-diethylanilid;
2-Ethyl-6-methyl-N-(1-methyl-2-methoxyethyl)-chloracetanilid;
2-Chlor-N-(2,6-dimethylphenyl)-N-[(1H)-pyrazol-1-yl-methyl]-acetamid;
α-Chlor-2',6'-diethyl-N-(2-propoxyethyl)-acetanilid;
N,N-Diisoproyl-S-(2,3,3,-trichlorallyl)-thiolcarbamat;
S-Ethyl-N,N-hexamethylen-thiolcarbamat;
2,6-Dinitro-4-trifluormethyl-N,N-dipropylanilin;
N-(1-Ethylpropyl)-3,4-dimethyl-2,6-dinitroanilin;
2-Chlor-N-{[(4-methoxy-6-methyl-1,3,5-triazin-2-yl)-amino]-carbonyl}-benzolsulfonamid;
2-{[[((4-Methoxy-6-methyl-1,3,5,-triazin-2-yl)-amino)-carbonyl]-amino]-sulfonyl}-benzoesäure oder deren Methylester;
Ethyl-2-{[(4-chlor-6-methoxy-2-pyrimidinyl)-aminocarbonyl]-aminosulfonyl}-benzoat;
2-[4-(2,4-Dichlorphenoxy)-phenoxy]-propionsäuremethylester;
2-{4-[(3-Chlor-5-trifluormethyl-2-pyridinyl)-oxy]-phenoxy}-propansäure bzw. -propansäureethylester;
Methyl-5-(2,4-dichlorphenoxy)-2-nitrobenzoat;
5-(2-Chlor-4-trifluormethyl-phenoxy)-2-nitro-benzoesäure;
3,5-Diiod-4-hydroxybenzonitril;
3,5-Dibrom-4-hydroxy-benzonitril;
2-[5-Methyl-5-(1-methylethyl)-4-oxo-2-imidazolin-2-yl]-3-chinolincarbonsäure;
Methyl-2-[4,5-dihydro-4-methyl-4-(1-methylethyl)-5-oxo-1H-imidazol-2-yl]-4(5)-methylbenzoat;
exo-1-Methyl-4-(1-methylethyl)-2-(2-methylphenyl-methoxy)-7-oxabicyclo-(2,2,1)-heptan;
2,4-Dichlorphenoxyessigsäure;
2,4-Dichlorphenoxypropionsäure;
(4-Chlor-2-methylphenoxy)-propionsäure;
(2-Methyl-4-chlorphenoxy)-essigsäure;
O-(6-Chlor-3-phenyl-pyridazin-4-yl)-S-octyl-thiocarbonat oder 3-Isopropyl-2,1,3-benzothiadiazin-4-on-2,2-dioxid sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken, Sie hängt im wesentlichen von der Art des gewünschten Effektes ab. Im allgemeinen liegen die Aufwandmengen zwischen 0,01

und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele:

Beispiel 1:

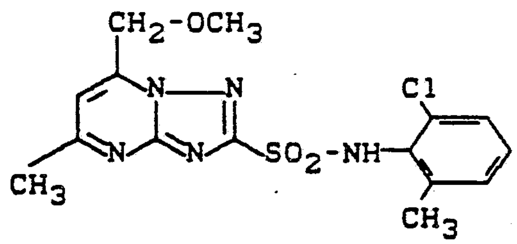

+

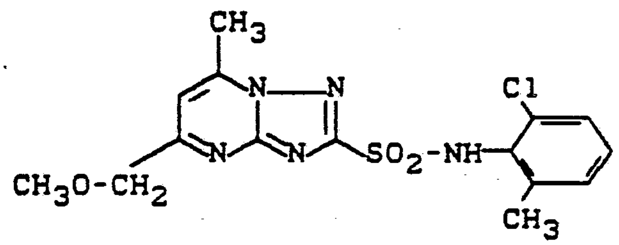

(Verfahren a)

5,0 g (0,0174 Mol) 5-Amino-1,2,4-triazol-3-yl-N-(2-chlor-6-methyl-phenyl)-sulfonamid und 2,9 g (0,0223 Mol) Methoxyacetylaceton werden in 30 ml Eisessig eine Stunde unter Rückfluß erhitzt. Die erkaltete Reaktionsmischung wird im Vakuum eingeengt und der Rückstand mit Ether verrührt. Der ausgefallene Feststoff wird abgesaugt und getrocknet. Man erhält 5,7 g (86 % der Theorie) eines Gemisches aus 5-Methyl-7-methoxymethyl-N-(2-chlor-6-methylphenyl)-1,2,4-triazolo-[1,5-a]-pyrimidin-2-sulfonamid (70 %) und 7-Methyl-5-methoxymethyl-N-(2-chlor-6-methyl-phenyl)-1,2,4-triazolo-[1,5-a]-pyrimidin-2-sulfon amid (30 %) vom Schmelzpunkt 203 °C.

Beispiel 2:

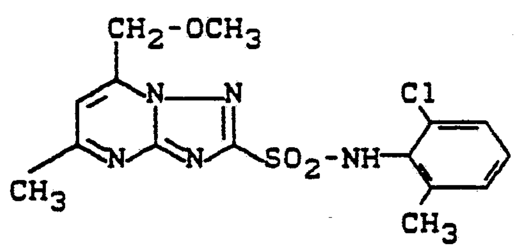

Aus der Mischung der stellungsisomeren Verbindungen (Beispiel 1) erhält man durch mehrmaliges Umkristallisieren 2,0 g (30 % der Theorie) an 5-Methyl-7-methoxymethyl-N-(2-chlor-6-methyl-phenyl)-1,2,4-triazolo-{1,5-a]-pyrimidin-2-sulfonamid vom Schmelzpunkt 227 °C.

Beispiel 3:

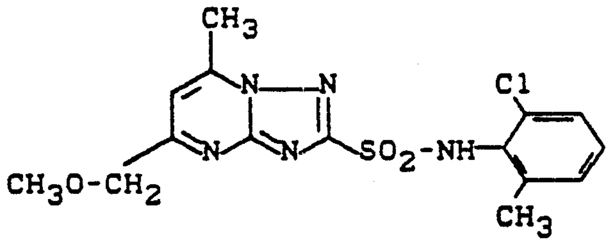

(Verfahren b)

Zu 1,35 g (0,00935 Mol) 2-Chlor-6-methylanilin, 0,75 g (0,0095 Mol) absolutem Pyridin und 0,12 g (0,001 Mol) 4-Dimethylaminopyridin in 50 ml trockenem Dichlormethan gibt man 2,7 g (ca. 0,0095 Mol) eines Rohprodukt-Gemisches aus 6-Chlor-5-methyl-7-methoxymethyl-1,2,4,-triazolo-[1,5-a]-pyrimidin-2-sulfochlorid und 7-Methyl-5 methoxymethyl-1,2,4,-triazolo-[1,5-a]-pyrimidin-2-sulfochlorid in 5 ml trockenem Dichlormethan, rührt 15 Stunden bei Raumtemperatur, wäscht mit Wasser, trocknet über Natriumsulfat und engt im Vakuum ein. Der ölige Rückstand wird chromatographisch getrennt (Keiselgel; Laufmittel : Dichlormethan/Aceton 4:1). Als 2. Fraktion erhält man 0,7 g (19 % der Theorie bezogen auf eingesetzte Vorstufe 5(7)-Methyl-7(5)-methoxymethyl-2-mercapto-1,2,4-triazolo-[1,5-a]-pyrimidin) an 7-Methyl-5-methoxymethyl-N-(2-chlor-6-methylphenyl)-1,2,4,-triazolo-[1,5-a] pyrimidin-2-sulfonamid vom Schmelzpunkt 169 °C.

Als Nebenprodukt eluiert man zunächst 0,5 g (12 % der Theorie bezogen auf eingesetzte Vorstufe 5(7)-Methyl-7(5)-methoxymethyl-2-mercapto-1,2,4-triazolo-[1,5-a]-pyrimidin) an 6-Chlor-5-methyl-7-methoxymethyl-N-(2-chlor-6-methylphenyl)-1,2,4-triazolo-[1,5-a]-pyrimidin-2 sulfonamid vom Schmelzpunkt 244 °C (Zers.).

In entsprechender Weise und gemäß den allgemeinen Angaben zur Herstellung erhält man die folgenden Triazolo-pyrimidin-2-sulfonamide der allgemeinen Formel (I):

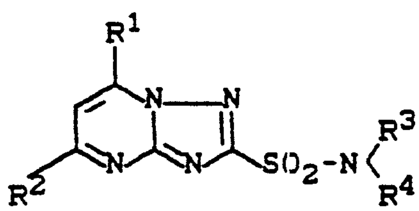

(I)

Tabelle 1

| Bsp. Nr. | $R^1$ | $R^2$ | $-N\langle^{R^3}_{R^4}$ | Schmelz-punkt/° C |
|---|---|---|---|---|
| 4 | $CH_3$ ($CH_3O-CH_2-$) | $CH_3O-CH_2-$ ($CH_3$) | 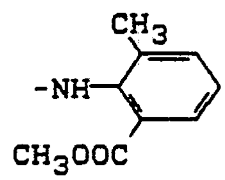 | Öl $^1H$-NMR*): 4,67 und 4,85 |
| 5 | $CH_3O-CH_2-$ | $CH_3$ | 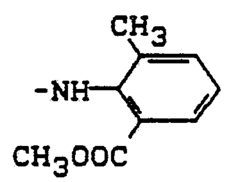 | 102-105 |
| 6 | $CH_3O-CH_2-$ | $CH_3$ | 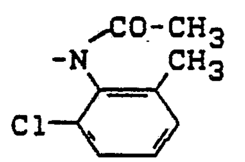 | 195 (Zers.) |
| 7 | $CH_3O-CH_2-$ | $CH_3$ | 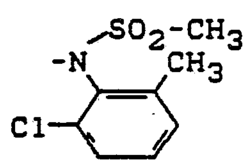 | 192 (Zers.) |
| 8 | $CH_3O-CH_2-$ | $CH_3$ | 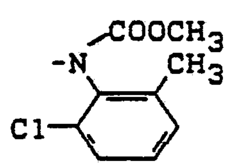 | 167 (Zers.) |
| 9 | $CH_3O-CH_2-$ | $CH_3$ | 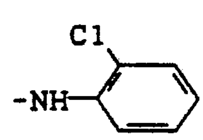 | 153-155 |

Tabelle 1

| Bsp. Nr. | $R^1$ | $R^2$ | $-N{<}^{R^3}_{R^4}$ | Schmelz-punkt /° C |
|---|---|---|---|---|
| 10 | $CH_3O-CH_2-$ | $CH_3$ | 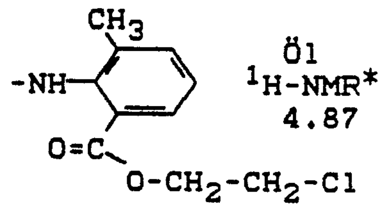 | Öl $^1H$-NMR*): 4.87 |
| 11 | $CH_3O-CH_2-$ | $CH_3$ | 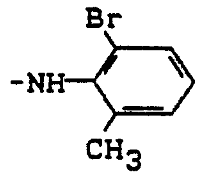 | 214-216 |

*) Die $^1H$-NMR-Spektren wurden in $CDCl_3$ mit TMS (Tetramethylsilan) als innerem Standard aufgenommen. Angegeben ist die chemische Verschiebung als δ-Wert in ppm.

Herstellung der Vorprodukte:

Beispiel II-1:

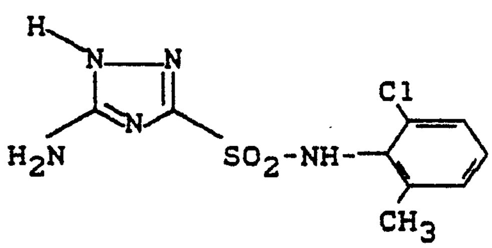

34,1 g (0,0837 Mol) 5-Amino-1-phenoxycarbonyl-1,2,4-triazol-3-yl-[N-(2-chlor-6-methyl-phenyl)]-sulfonamid in 350 ml Ethanol werden mit 14,9 g (0,168 Mol) 45prozentiger wässriger Natronlauge versetzt, eine Stunde bei Raumtemperatur gerührt, mit Eisessig angesäuert und im Vakuum eingeengt. Der Rückstand wird dreimal mit Wasser gewaschen und anschließend bei 80 °C im Vakuum getrocknet. Der so erhaltenen Feststoff wird durch Verrühren mit Diethylether gereinigt.

Man erhält 13,5 g (56 % der Theorie) an 5-Amino-1,2,4-triazol-3-yl-[N-(2-chlor-6-methyl-phenyl)]-sulfonamid vom Schmelzpunkt 217 °C.

In analoger Weise erhält man die folgenden Verbindungen:

Beispiel II-2:

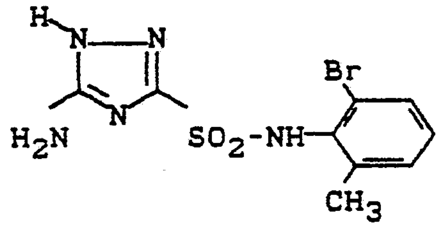

Fp. 137° -139° C

Beispiel II-3:

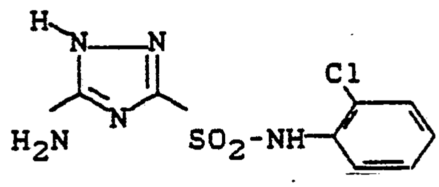

Fp 176° -180° C

Beispiel II-4:

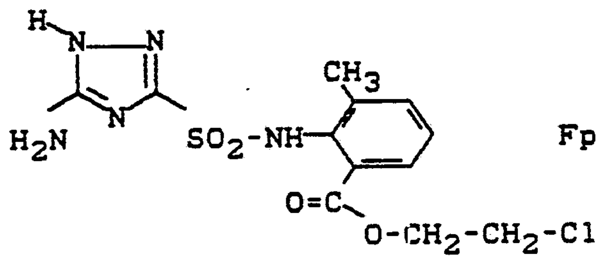

Fp.224° -226° C

Beispiel X-1:

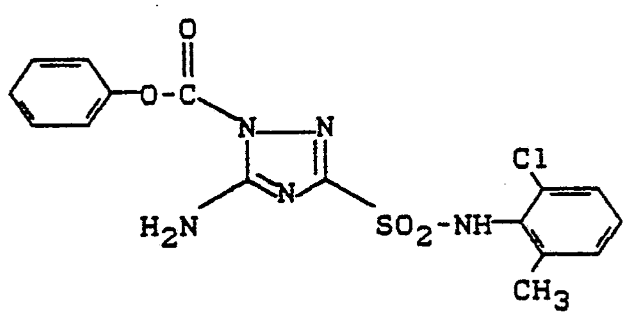

Zu 7,3 g (0,0922 Mol) absolutem Pyridin, 13 g (0,092 Mol) 2-Chlor-6-methylanilin und 1,1 g (0,009 Mol) 4-Dimethylaminopyridin im 300 ml trockenem Dichlormethan gibt man 27,9 g (0,0922 Mol) 5-Amino-1-phenoxycarbonyl-1,2,4-triazol-3-yl-sulfochlorid, rührt eine Stunde bei Raumtemperatur, entfernt das Lösungsmittel im Vakuum, verrührt den öligen Rückstand mit Wasser, dekantiert und kristallisiert das verbleibende Öl aus Ethanol.

Man erhält 35,5 g (95 % der Theorie) an 5-Amino-1-phenoxycarbonyl-1,2,4-triazol-3-yl-[N-(2-chlor-6-methylphenyl)]-sulfonamid von Schmelzpunkt 200 °C.

In analoger Weise erhält man die folgenden Verbindungen:

Besipiel X-2:

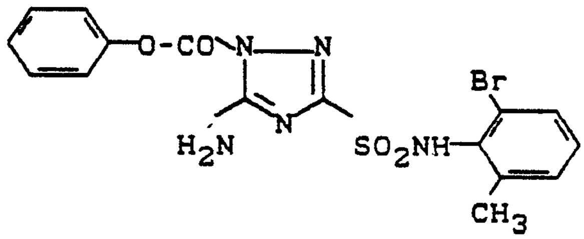

Fp. 186° C

Beispiel X-3:

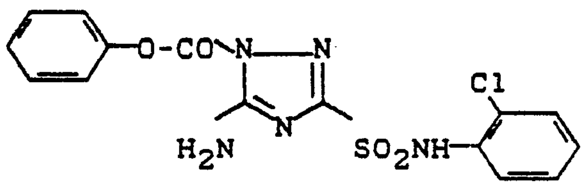

Fp. 176-178° C

Beispiel X-4:

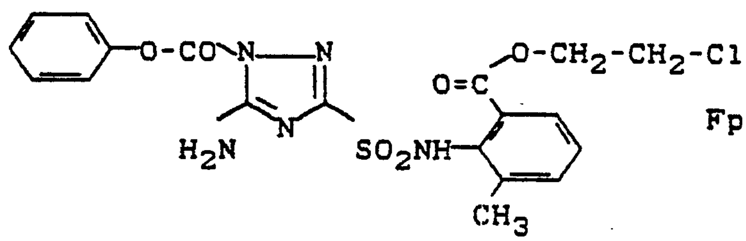

Fp. 176-178° C

Beispiel IX:

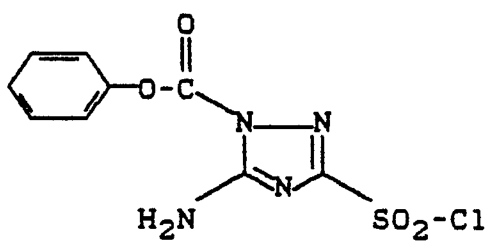

Zu 136,6 g (0,419 Mol) 5-Amino-1-phenoxycarbonyl-3-benzylthio-1,2,4-triazol in 1700 ml Chloroform gibt man 30 ml Eisessig und 15 ml Wasser und leitet durch diese Suspension bei - 5° C eine Stunde lang einen nicht getrockneten Chlorgasstrom, wobei sich eine klare Lösung bildet.

Zu Aufarbeitung entfernt man das Lösungsmittel im Vakuum und verrührt den Rückstand mit 1 l Ether. Der etherunlösliche Feststoff wird abgesaugt und getrocknet.

Man erhält 101 g (80 % der Theorie) an 5-Amino-1-phenoxycarbonyl-1,2,4-triazol-3-yl-sulfochlorid vom Schmelzpunkt 164 °C.

Beispiel VIII:

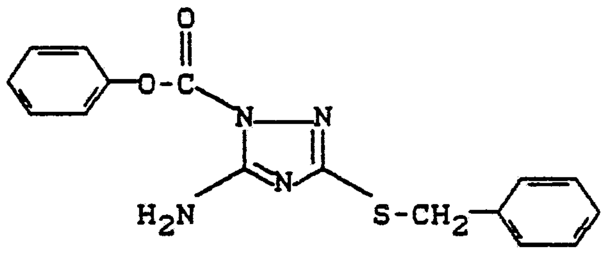

Zu 100 g (0,4854 Mol) 3-Amino-5-benzylthio-1,2,4-triazol (vgl. J. Het. Chem. 12, 1187 [1975]) in 700 ml absolutem Pyridin tropft man unter Kühlung 83,6g (0,5339 Mol) Chlorameisensäurephenylester, so daß die Innentemperatur nicht über 5 °C ansteigt. Nach beendeter Zugabe rührt man weitere 60 Minuten bei 10 °C, gießt dann das Reaktionsgemisch in Eiswasser, saugt den ausgefallenen Feststoff ab und kristallisiert aus Ethanol um.

Man erhält 140 g (88,5 % der Theorie) an 5-Amino-3-benzylthio-1-phenoxycarbonyl-1,2,4-triazol vom Schmelzpunkt 285 °C (Zers.).

Beispiel IV-1/XII-1:

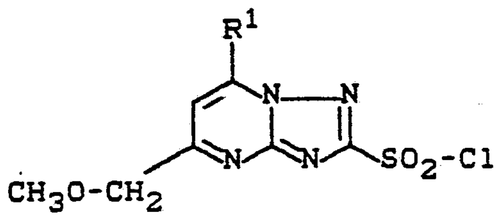

(IV-1)

+

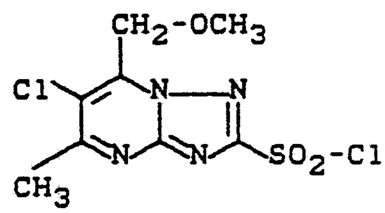

(XII-1)

Zu 2 g (0,0095 Mol) eines 1:1-Gemisches aus 5-Methyl-7-methoxymethyl-2-mercapto-1,2,4-triazolo-[1,5-a]-pyrimidin und 7-Methyl-5-methoxymethyl-2-mercapto-1,2,4-triazolo-[1,5-a]-pyrimidin in 150 ml Chloroform gibt man 0,5 ml Wasser und leitet durch diese Suspension bei -5 °C ca. 30 Minuten einen nicht getrockneten Chlorgasstrom, wobei sich eine klare Lösung bildet. Zur Aufarbeitung entfernt man das Lösungsmittel im Vakuum und setzt das so erhaltene farblose Öl ohne weitere Reinigung in die nächste Stufe ein.

Beispiel XIa-1/XIb-1:

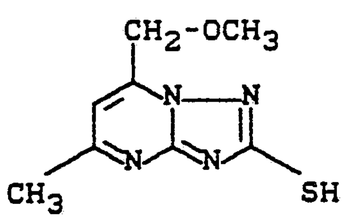

+

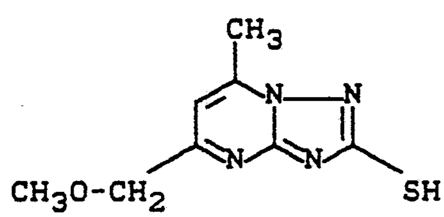

10,0 g (0,086 Mol) 3-Amino-5-mercapto-1,2,4-triazol und 13,4 g (0,103 Mol) Methoxyacetylaceton in 100 ml Eisessig werden 90 Minuten unter Rückfluß erhitzt. Aus der erkalteten Reaktionsmischung fällt das Produkt in kristalliner Form aus.

Man erhält 12 g (66 % der Theorie) einer Mischung aus 5-Methyl-7-methoxymethyl-3-mercapto-1,2,4-triazolo-[1,5-a]-pyrimidin ( 50 %) und 7-Methyl-5-methoxymethyl-3-mercapto-1,2,4-triazolo-[1,5a]-pyrimidin

(50 %) vom Schmelzpunkt 209 °C.

Anwendungsbeispiele:

In den folgenden Anwendungsbeispielen wurde die nachstehend aufgeführte Verbindung als Vergleichsubstanz eingesetzt:

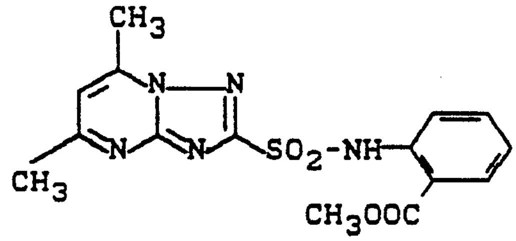

(A)

5,7-Dimethyl-N-(2-methoxycarbonyl-phenyl)-1,2,4-triazolo-[1,5-a]-pyrimidin-2-sulfonamid

(bekannt aus EP 142 152/Verbindung 10)

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

Eine deutliche Überlegenheit in der herbiziden Wirksamkeit gegen mono-und dikotyle Unkräuter gegenüber der Vergleichsverbindung (A) zeigen in diesem Test beispielsweise die Verbindungen gemäß den Herstellungsbeispielen 1, 2, 3, 4, 5, 6, 7 und 8.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das

Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)

100 % = totale Vernichtung

Eine deutliche Überlegenheit in der herbiziden Wirksamkeit gegen mono-und dikotyle Unkräuter gegenüber der Vergleichsverbindung (A) zeigen in diesem Test z.B. die Verbindungen gemäß den Herstellungsbeispielen 1, 2, 3, 4, 5, 6, 7 und 8.

## Ansprüche

1. Triazolo-pyrimidin-2-sulfonamide der Formel (I),

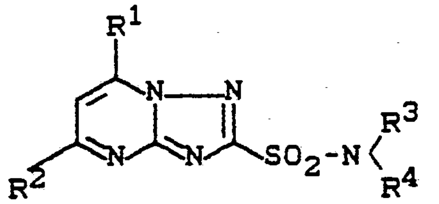

(I)

in welcher

$R^1$ für einen Rest $-CH_2-O-R^5$ steht und gleichzeitig $R^2$ für Alkyl steht oder

$R^2$ für einen Rest $-CH_2-O-R^5$ steht und gleichzeitig $R^1$ für Alkyl steht,

$R^3$ für Wasserstoff, Alkyl, Alkylcarbonyl, Alkoxycarbonyl, Alkylsulfonyl, Alkenyl, Alkinyl oder für gegebenenfalls substituiertes Aralkyl steht,

$R^4$ für jeweils gegebenenfalls substituiertes Aryl oder Heteroaryl steht und

$R^5$ für Alkyl steht.

2. Triazolo-pyrimidin-2-sulfonamide der Formel (I) gemäß Anspruch 1, in welcher

$R^1$ für einen Rest $-CH_2-O-R^5$ steht und gleichzeitig $R^2$ für geradkettiges oder verzweigtes Alkyl mit 1 bis 6 Kohlenstoffatomen steht oder

$R^2$ für einen Rest $-CH_2-O-R^5$ steht und gleichzeitig $R^1$ für geradkettiges oder verzweigtes Alkyl mit 1 bis 6 Kohlenstoffatomen steht, wobei

$R^5$ in beiden Fällen jeweils für geradkettiges oder verzweigtes Alkyl mit 1 bis 6 Kohlenstoffatomen steht,

$R^3$ für Wasserstoff, für jeweils geradkettiges oder verzweigtes Alkyl, Alkylcarbonyl, Alkoxycarbonyl oder Alkylsulfonyl mit jeweils 1 bis 4 Kohlenstoffatomen in den einzelnen Alkylteilen, für jeweils geradkettiges oder verzweigtes Alkenyl oder Alkinyl mit jeweils 3 bis 6 Kohlenstoffatomen oder für im Alkylteil geradkettiges oder verzweigtes und im Arylteil gegebenenfalls einfach oder mehrfach, gleich oder verschieden substituiertes Aralkyl mit 1 bis 4 Kohlenstoffatomen im Alkylteil und 6 bis 10 Kohlenstoffatomen im Arylteil steht, wobei als Arylsubstituenten infrage kommen:

Fluor, Chlor, Brom, Iod, Cyano, Nitro, Hydroxy sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen und

$R^4$ für gegebenenfalls einfach oder mehrfach, gleich oder verschieden substituiertes Aryl mit 6 bis 10 Kohlenstoffatomen oder für einen gegebenenfalls einfach oder mehrfach, gleich oder verschieden substituierten und/oder benzannelierten 5-bis 7-gliedrigen Heterocyclus mit 1 bis 3 Heteroatomen, insbesondere Stickstoff, Sauerstoff und/oder Schwefel steht, wobei als Substituenten jeweils infrage kommen: Fluor, Chlor, Brom, Iod, Cyano, Nitro, Hydroxy, jeweils geradkettiges oder verzweigtes Alkyl, Alkoxy, Alkylthio, Alkylcarbonyl, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl, Halogenalkylsulfonyl, Halogenalkylcarbonyl oder Halogenalkoxycarbonyl mit jeweils 1 bis 6 Kohlenstoffatomen und 1 bis 9 gleichen oder verschiedenen Halogenatomen, Phenyl, Phenoxy, Phenylthio, Phenylcarbonyl, Hydroxycarbonyl, jeweils geradkettiges oder verzweigtes Alkoxycarbonyl, Alkenyloxycarbonyl oder Alkoxyalkoxycarbonyl mit

jeweils 1 bis 6 Kohlenstoffatomen in den einzelnen Alkylteilen bzw. 3 bis 6 Kohlenstoffatomen im Alkenylteil, sowie Hydroximinoalkyl oder geradkettiges oder verzweigtes Alkoximinoalkyl mit jeweils 1 bis 4 Kohlenstoffatomen in den einzelnen Alkylteilen.

3. Verfahren zur Herstellung von Triazolo-pyrimidin-2-sulfonamiden der allgemeinen Formel (I)

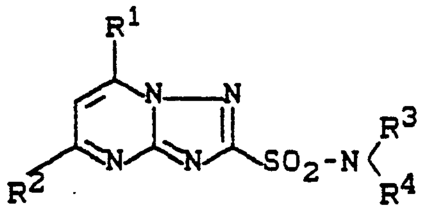

(I)

in welcher
$R^1$ für einen Rest $-CH_2-O-R^5$ steht und gleichzeitig $R^2$ für Alkyl steht oder
$R^2$ für einen Rest $-CH_2-O-R^5$ steht und gleichzeitig $R^1$ für Alkyl steht,
$R^3$ für Wasserstoff, Alkyl, Alkylcarbonyl, Alkoxycarbonyl, Alkylsulfonyl, Alkenyl, Alkinyl oder für gegebenenfalls substituiertes Aralkyl steht und
$R^4$ für jeweils gegebenenfalls substituiertes Aryl oder Heteroaryl steht und
$R^5$ für Alkyl steht,
dadurch gekennzeichnet, daß man

(a) Triazolo-pyrimidin-2-sulfonamide der Formel (Ia)

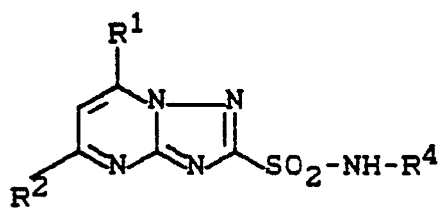

(Ia)

in welcher
$R^1$, $R^2$ und $R^4$ die oben angegebene Bedeutung haben,
erhält, wenn man Amino-triazolylsulfonamide der Formel (II),

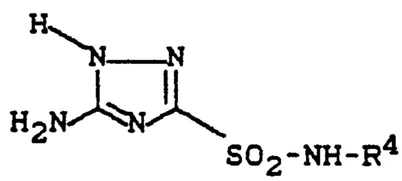

(II)

in welcher
$R^4$ die oben angegebene Bedeutung hat,
mit 1,3 -Diketonen der Formel (III),

$$R^6-\overset{O}{\overset{\|}{C}}-CH_2-\overset{O}{\overset{\|}{C}}-CH_2-O-R^5 \qquad (III)$$

in welcher
$R^5$ die oben angegebene Bedeutung hat und
$R^6$ für Alkyl steht,
gegebenenfalls in Gegenwart eines Verdünnungsmittels umsetzt;
oder daß man

(b) Triazolo-pyrimidin-2-sulfonamide der Formel (Ib),

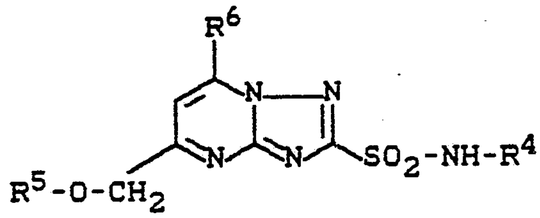

(Ib)

in welcher
$R^4$ und $R^5$ die oben angegebene Bedeutung haben und
$R^6$ für Alkyl steht,
erhält, wenn man Triazolo-pyrimidin-2-sulfochloride der Formel (IV),

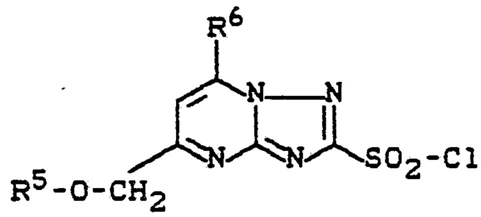

(IV)

in welcher
$R^5$ und $R^6$ die oben angegebene Bedeutung haben,
mit Aminen der Formel (V),
$R^4$-$NH_2$ (V)
in welcher
$R^4$ die oben angegebene Bedeutung hat,
gegebenenfalls in Gegenwart eines Verdünnungsmittels und gegebenenfalls in Gegenwart eines Säurebindemittels umsetzt;
oder daß man
(c) Triazolo-pyrimidin-2-sulfonamide der Formel (Ic),

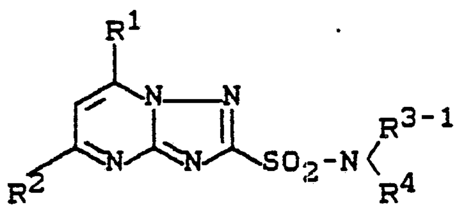

(Ic)

in welcher
$R^1$, $R^2$ und $R^4$ die oben angegebene Bedeutung haben und
$R^{3-1}$ für Alkyl, Alkenyl, Alkinyl, für gegebenenfalls substituiertes Aralkyl, für Alkylcarbonyl, Alkoxycarbonyl oder Alkylsulfonyl steht,
erhält, wenn man die nach Verfahren (a) oder (b) erhältlichen Triazolo-pyrimidin-2-sulfonamide der Formel (Ia)

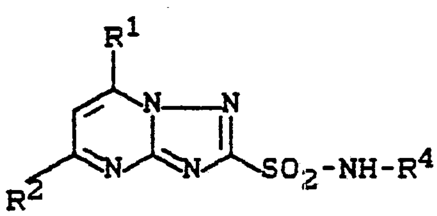

(Ia)

in welcher
$R^1$, $R^2$ und $R^4$die oben angegebene Bedeutung haben,
mit Alkylierungs-, Acylierungs-, oder Sulfonylierungsmitteln der Formel (VI),

$R^{3-1}$-Y (VI),
in welcher
$R^{3-1}$ die oben angegebene Bedeutung hat und
Y für eine elektronenanziehende Abgangsgruppe steht,
gegebenenfalls in Gegenwart eines Verdünnungsmittels und gegebenenfalls in Gegenwart einer Base umsetzt.

4. Herbizide Mittel, gekennzeichnet durch einen Gehalt an mindestens einem Triazolo-pyrimidin-2-sulfonamid der Formel (I) gemäß den Ansprüchen 1 bis 3.

5. Verwendung von Triazolo-pyrimidin-2-sulfonamiden der Formel (I) gemäß den Ansprüchen 1 bis 3 zur Bekämpfung von Umkräutern.

6. Verfahren zur Herstellung von herbiziden Mitteln, dadurch gekennzeichnet, daß man Triazolo-pyrimidin-2-sulfonamide der Formel (I) gemäß den Ansprüchen 1 bis 3 mit Streckmitteln und/oder oberflächenaktiven Substanzen vermischt.

7. Aminotriazolylsulfonamide der Formel (II),

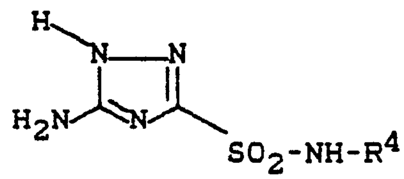

(II)

in welcher
$R^4$ für jeweils gegebenenfalls substituiertes Aryl oder Heteroaryl steht.

8. Triazolo-pyrimidin-2-sulfochloride der Formel (IV)

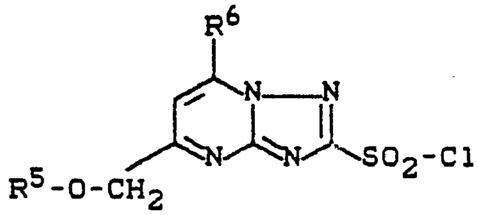

(IV)

in welcher
$R^5$ für Alkyl steht und
$R^6$ für Alkyl steht.

9. Triazolopyrimidin-Derivate der Formeln (XIa) und (XIb)

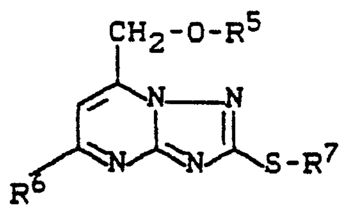

und

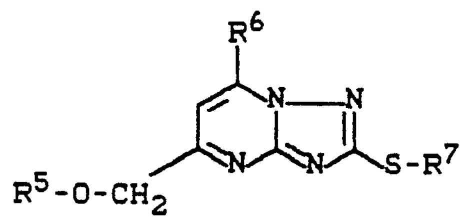

(XIa) (XIb)

inwelcher
$R^5$ für Alkyl steht,
$R^6$ für Alkyl steht und
$R^7$ für Wasserstoff oder einen Benzylrest steht.

10. Verbindungen der Formel (XIV),

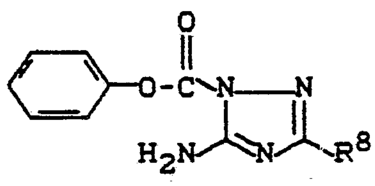

(XIV)

in welcher
$R^8$ für Benzylthio, $-SO_2Cl$ oder $-SO_2-NH-R^4$ steht, wobei
$R^4$ für jeweils gegebenenfalls substituiertes Aryl oder Heteroaryl steht.

11. Verbindung der Formel